# EUROPEAN PATENT APPLICATION

(11) **EP 3 831 315 A2**
(43) Date of publication of application: **09.06.2021**
(21) Application number: 20211927.7
(22) Date of filing: 04.12.2020
(51) Int. Cl.: A61B 17/115

(54) **SURGICAL STAPLING INSTRUMENTS**

(30) Priority: 06.12.2019 US 201962944548 P; 05.11.2020 US 202017089813
(71) Applicant: Covidien LP, Mansfield, MA 02048 (US)
(72) Inventor: NICHOLAS, David A., Trumbull, CT 06611 (US); PRIBANIC, Russell, Roxbury, CT 06783 (US)
(74) Representative: Maschio, Antonio

(57) **Abstract**

A surgical stapling instrument includes an elongated handle housing, an elongated body portion extending distally from the handle housing, and an end effector coupled to the elongated body portion and configured to perform an anastomosis. A screw is disposed within the handle housing and has a distal end portion configured to close the end effector about tissue. A rotatable knob is supported by the handle housing and coupled to a proximal end portion of the screw, such that a rotation of the knob results in axial motion of the screw.

## Description

### CROSS-REFERENCE TO RELATED APPLICATION

This application claims the benefit of and priority to U.S. Provisional Patent Application No. 62/944,548, filed December 6, 2019, the entire disclosure of which is incorporated by reference herein.

### BACKGROUND

### 1. Technical Field

The present disclosure is directed to surgical instruments, such as surgical stapling instruments. In particular, the present disclosure relates to hand-held, battery-operated circular stapling instruments.

### 2. Background of Related Art

Circular stapling instruments are used to perform end-to-end anastomosis procedures within a patient. During an end-to-end anastomosis procedure, an end of a first vessel portion is joined to an end of a second vessel portion. Typically, circular stapling instruments include an anvil, which defines an annular array of staple deforming depressions, and an annular cartridge housing annular rows of staples. During actuation of the circular stapling instrument, the anvil is approximated toward the annular cartridge to clamp tissue therebetween. When it is determined that the tissue has been clamped between the anvil and the annular cartridge, staples may then be ejected into the clamped tissue.

### SUMMARY

In one aspect of the present disclosure, a surgical stapling instrument is provided. The surgical stapling instrument includes an elongated handle housing, a screw disposed within the handle housing, a rotatable knob supported by the handle housing, an elongated body portion extending distally from the handle housing, and an end effector. The knob is coupled to a proximal end portion of the screw, such that a rotation of the knob results in motion of the screw. The end effector includes a cartridge assembly coupled to a distal end portion of the elongate body portion, and an anvil assembly coupled to a distal end portion of the screw. The anvil assembly is configured to translate relative to the cartridge assembly in response to a manual rotation of the knob to move the end effector between unapproximated and approximated positions.

In some aspects, the knob may extend proximally from a proximal end portion of the handle housing.

In some aspects, the knob, the screw, and a proximal end portion of the elongated body portion may be coaxial.

In some aspects, the screw may be configured to translate within the handle housing in response to a rotation of the knob.

In some aspects, the surgical stapling instrument may further include a sensor configured to determine a relative axial position of the screw.

In some aspects, the screw may have a flag extending outwardly therefrom. The flag may be disposed adjacent the sensor, such that the sensor is configured to determine a relative axial position of the flag.

In some aspects, the surgical stapling instrument may further include a fire switch and a light disposed adjacent the fire switch. The fire switch may be coupled to the handle housing and in communication with a motor. The sensor may be configured to turn on the light upon determining that the screw is in an axial position corresponding to the approximated position of the end effector.

In some aspects, the surgical stapling instrument may further include an instrument module insertable into a cavity defined by the handle housing. The instrument module may include a housing, a motor disposed within the housing, and an output gear drivingly coupled to the motor.

In some aspects, the surgical stapling instrument may further include a nut gear operably coupled to the output gear, and a fire shaft having a proximal end portion and a distal end portion. The proximal end portion of the fire shaft may be operably coupled to the nut gear, and the distal end portion of the fire shaft may be coupled to a pusher of the cartridge assembly. The fire shaft may be configured to translate the pusher in response to a rotation of the nut gear.

In some aspects, the nut gear may have a plurality of gear teeth disposed about an outer periphery thereof. The nut gear may further include a threaded inner surface threadedly engaged to the proximal end portion of the fire shaft.

In some aspects, the screw may extend longitudinally through the fire shaft and the nut gear.

In some aspects, the surgical stapling instrument may further include a fire gear intercoupling the output gear and the nut gear.

In accordance with another aspect of the present disclosure, a handle assembly of a circular stapler is provided. The handle assembly includes an elongated handle housing, a drive shaft disposed within the handle housing, and a rotatable knob supported by the handle housing. The drive shaft has a distal end portion configured to effect an opening and closing of an end effector. The knob is coupled to a proximal end portion of the drive shaft, such that a rotation of the knob results in axial motion of the drive shaft.

In some aspects, the knob may extend proximally from a proximal end portion of the handle housing.

In some aspects, the drive shaft may be a screw disposed within a bottom end portion of the handle housing.

In some aspects, the handle assembly may further include a sensor disposed in the handle housing. The drive shaft may have a flag extending outwardly therefrom. The flag may be disposed adjacent the sensor, such that the sensor is configured to determine a relative axial position of the flag.

In some aspects, the handle assembly may further include a fire switch and a light disposed adjacent the fire switch. The fire switch may be coupled to the handle housing and in communication with a motor. The sensor may be configured to turn on the light upon determining that the drive shaft is in an axial position corresponding to an approximated position of the end effector.

In some aspects, the handle assembly may further include an instrument module insertable into a cavity defined by the handle housing.

In some aspects, the handle assembly may further include a nut gear operably coupled to the output gear, and a fire shaft. The fire shaft may have a proximal end portion operably coupled to the nut gear, and a distal end portion configured to effect a stapling function of the end effector.

In some aspects, the drive shaft may extend longitudinally through the fire shaft and the nut gear.

### BRIEF DESCRIPTION OF THE DRAWINGS

Embodiments of the presently disclosed surgical stapling instruments are disclosed herein, with reference to the following drawings:
FIG. 1 is a perspective view of a surgical stapling instrument including a handle assembly, an elongated body portion, and an end effector;
FIG. 2 is a perspective view, with a housing half of a handle housing removed, illustrating the surgical stapling instrument of FIG. 1 with an instrument module separated from the handle housing;
FIG. 3 is a side view of the surgical stapling instrument of FIG. 2 illustrating internal components of the handle assembly;
FIG. 4 is a longitudinal cross-sectional view of the surgical stapling instrument of FIG. 2, as taken through 4-4 of FIG. 2;
FIG. 5 is an enlarged, perspective view of the surgical stapling instrument, with the housing half removed, illustrating the internal components of the handle assembly;
FIG. 6 is an enlarged, perspective view, illustrating a fire switch and illumination lights of the handle assembly;
FIG. 7 is an enlarged view of the area of detail labeled "7" in FIG. 4; and
FIG. 8 is an enlarged, perspective view, illustrating the instrument module of FIG. 2 being connected to a printed circuit board of the handle assembly.

### DETAILED DESCRIPTION

Persons having skill in the art will understand the present disclosure from reading the following description in conjunction with the accompanying drawings. Reference characters indicate the same or similar elements throughout the drawings. As is customary, the term "distal" refers to a location farther from the user of the instrument and the term "proximal" refers to a location that is closer to the user of the instrument.

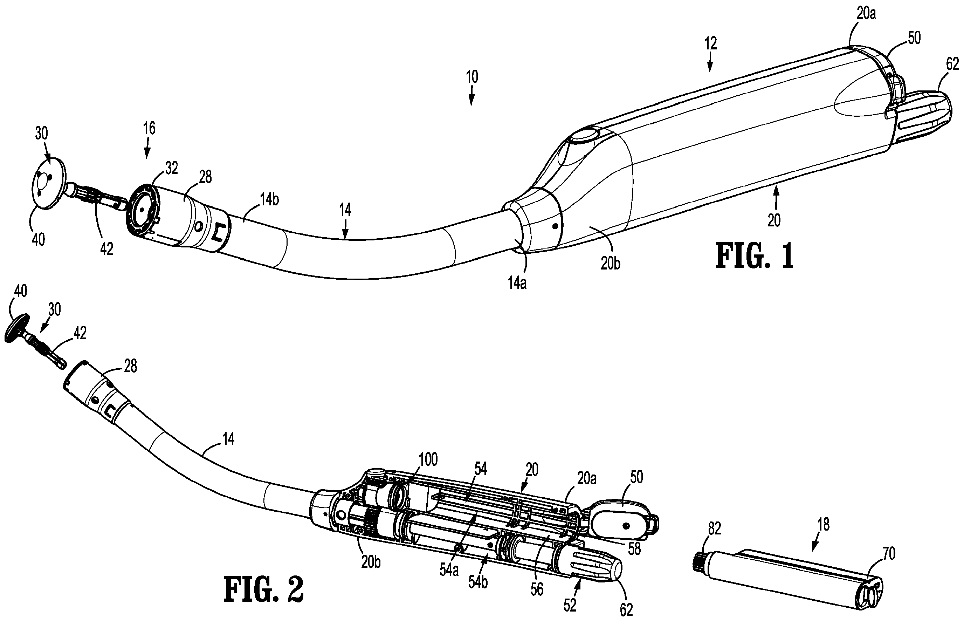

FIGS. 1 and 2 illustrate an embodiment of a surgical stapling instrument according to the present disclosure, referenced generally as a circular stapler 10. Circular stapler 10 includes a handle assembly 12, an elongated body portion 14, which is supported by and extends distally from the handle assembly 12, and an end effector 16 coupled to a distal end portion 14b of the elongated body portion 14. The handle assembly 12 includes an instrument or power module 18 insertable into a handle housing 20 of the handle assembly 12. The instrument module 18 is reusable, whereas the remainder of the circular stapler 10 (e.g., the handle housing 20, the elongated body portion 14, and the end effector 16) may be disposable. In aspects, the entire circular stapler 10 may be reusable or disposable.

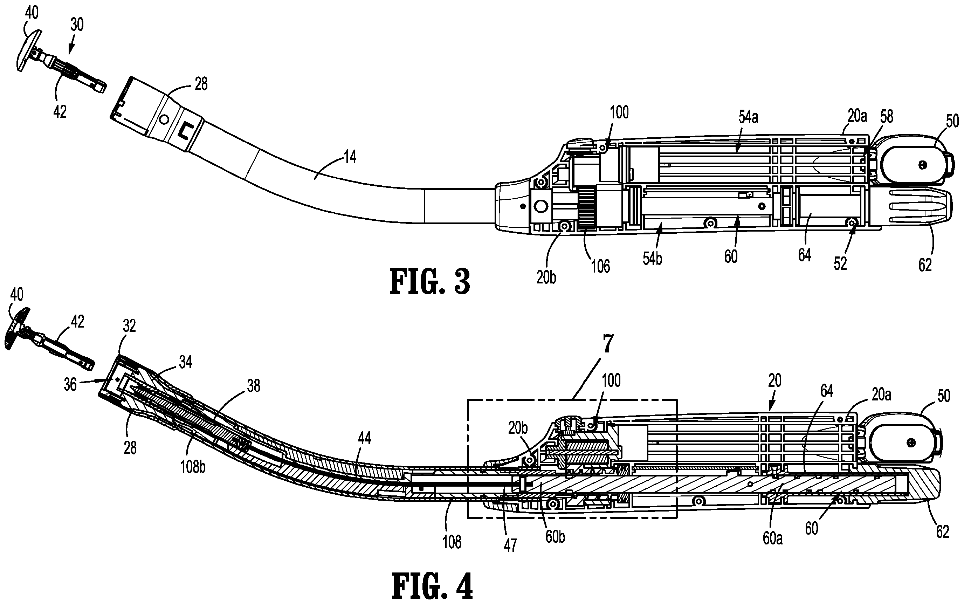

With reference to FIGS. 1-4, the end effector 16 is supported on the distal end portion 14b of the elongated body portion 14 or otherwise coupled to the distal end portion 14b of the elongated body portion 14. In some embodiments, the end effector 16 may be monolithically formed with or integrally connected to the distal end portion 14b of the elongated body portion 14. The end effector 16 includes a cartridge assembly 28 and an anvil assembly 30. The cartridge assembly 28 includes a staple cartridge 32 configured for supporting a plurality of surgical staples (not shown) therein and to discharge the staples into tissue after approximation of the cartridge assembly 28 and the anvil assembly 30. The staple cartridge 32 accommodates an annular pusher 34 (FIG. 4) for advancing the staples through the staple cartridge 32. The staple cartridge 32 defines a longitudinal channel 36 for movable receipt of a trocar 38 (FIG. 4) of the anvil assembly 30. The staple cartridge 32 has a plurality of annular rows of staple retaining recesses having the surgical staples disposed therein.

The anvil assembly 30 includes, inter alia, the trocar 38, an anvil head 40, and an anvil center rod 42 extending from the anvil head 40. The trocar 38 extends through and from the elongated body portion 14 and is movably disposed within the channel 36 of the cartridge assembly 28. The trocar 38 is configured to detachably (or in some aspects permanently) receive the anvil center rod 42 therein. A proximal end of the trocar 38 is configured to be removably or non-removably coupled to a central shaft 44 that extends longitudinally through the elongated body portion 14. The central shaft 44 is operable to selectively longitudinally move the trocar 38 to move the anvil head 40 between unapproximated and approximated positions, in relation to the cartridge assembly 28, in response to actuation of the handle assembly 12, to clamp tissue between the cartridge and anvil assemblies 28, 30, as will be described in further detail below.

Reference may be made to U.S. Patent No. 7,802,712 for a detailed description of the construction and operation of an end effector including a cartridge assembly and an anvil assembly similar to that disclosed herein, the entire contents of which being incorporated by reference herein.

With continued reference to FIGS. 1-4, the handle assembly 12 of the circular stapler 10 includes the handle housing 20, a door 50, and a clamping actuation assembly 52 for translating the central shaft 44 along with the attached anvil assembly 30. The handle housing 20 has an elongated configuration, and in some aspects, a generally rectangular configuration, and defines an elongated cavity 54 therein dimensioned for receipt of various components of the circular stapler 10. The cavity 54 has an upper end portion or half 54a for accommodating the instrument module 18, and a bottom end portion or half 54b separated from the upper end portion 54a by a partition 56. The handle housing 20 has a proximal end portion 20a defining an opening 58 dimensioned for removable receipt of the instrument module 18. The door 50 is hingedly coupled to the proximal end portion 20a of the handle housing 20 and configured to pivot between an opened position (FIG. 2), in which the proximal opening 58 of the handle housing 20 is exposed, and a closed position (FIG. 1), in which the door 50 covers the proximal opening 58 to enclose the instrument module 18 in the cavity 54.

The clamping actuation mechanism 52 of the handle assembly 12 includes a drive shaft 60 and a rotatable knob 62 coupled to the drive shaft 60. The drive shaft 60 may be a screw and extends through the bottom end portion 54b of the cavity 54 and in coaxial alignment with a longitudinal axis "X" defined by the elongated body portion 14. The screw 60 has a threaded proximal end portion 60a disposed in the proximal end portion 20a of the handle housing 20, and a distal end portion 60b disposed in a distal end portion 20b of the handle housing 20. The distal end portion 60b of the screw 60 is fixed to a proximal end portion 47 of the central shaft 44, such that the screw 60 and the central shaft 44 move longitudinally with one another.

The knob 62 of the clamping actuation mechanism 52 is coupled to the proximal end portion 60a of the screw 60. In aspects, an elongated collar 64 is provided and is rotationally supported in the proximal end portion 20a of the handle housing 20 and threadedly coupled to the threaded proximal end portion 60a of the screw 60. The knob 62 is fixed about the elongated collar 64 and protrudes proximally from the proximal end portion 20a of the handle housing 20. As such, a manual rotation of the knob 62 drives a translation of the screw 60 via the threaded engagement of the elongated collar 64 and the screw 60. In aspects, the knob 62 may be directly threadedly coupled to the screw 60. The knob 62 allows for the deliberate and precise clamping of tissue by the clinician. In some aspects, instead of using the manually-rotatable knob 62 for closing the end effector 16, a motor (not shown) may be provided to effect a closing of the end effector 16.

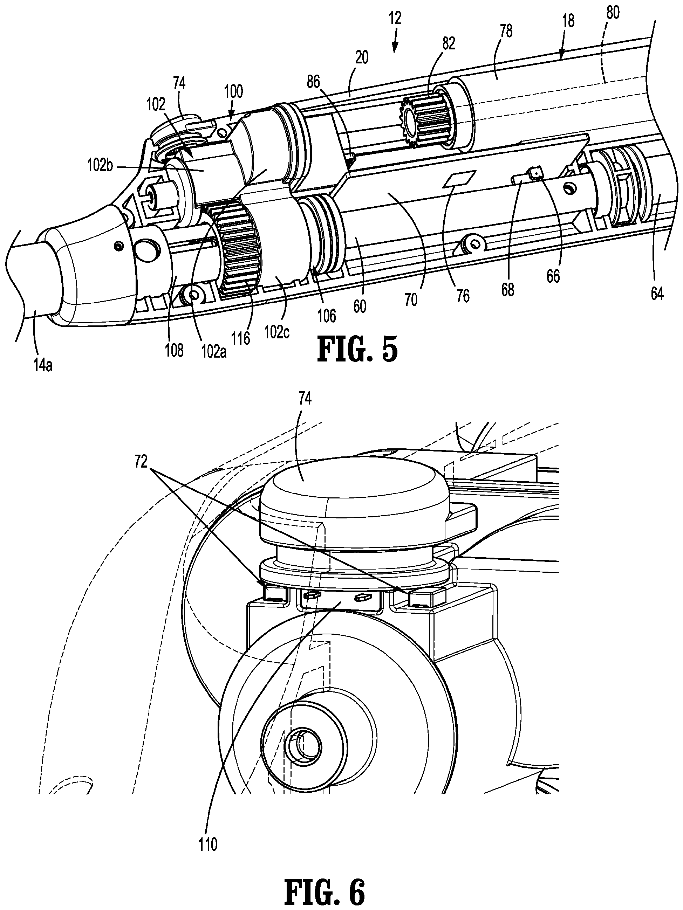

With reference to FIGS. 5 and 6, the handle assembly 12 includes an anvil position sensor 66, such as, for example, an optical sensor, for determining when the end effector 16 is in a clamped state and ready for firing. The screw 60 of the clamping actuation mechanism 52 has a flag or tab 68 extending radially outward therefrom. The flag 68 is disposed in side-by-side relation with the sensor 66 and moves with the screw 60 as the screw 60 effects an opening or closing of the end effector 16. The sensor 66 is configured to determine a relative axial position of the flag 68 of the screw 62 throughout the translation of the screw 60.

The sensor 66 may be supported on a first printed circuit board 70 of the handle assembly 12 and in electrical communication with one or more lights 72, such as, for example, LEDs, disposed adjacent a fire button 74. In aspects, the sensor 66 may be in wireless communication with a second printed circuit board 88, which is powered by the instrument module 18. The first or second printed circuit board 70 or 88 may have a processor 76 and a memory for storing instructions. The processor 76 is in electrical communication with the sensor 66 by wires, leads, or via wireless connection. The processor 76 is configured to send a signal to the lights 72 to turn on when the sensor 66 determines that the flag 68 of the screw 60 is disposed at a proximal-most position corresponding to an approximated/clamped state of the end effector 16. The lights 72 may be disposed underneath the fire button 74 to illuminate the fire button 74, thereby indicating to a clinician that the circular stapler 10 is ready for firing.

The processor 76 may be a controller, such as, for example, a microcontroller. The processor 76 may be an integrated circuit, analog or logic circuitry, and/or microprocessor, or an array of such components. The processor 76 receives information from the memory. In one embodiment, the memory may be an erasable programmable read only memory ("EPROM").

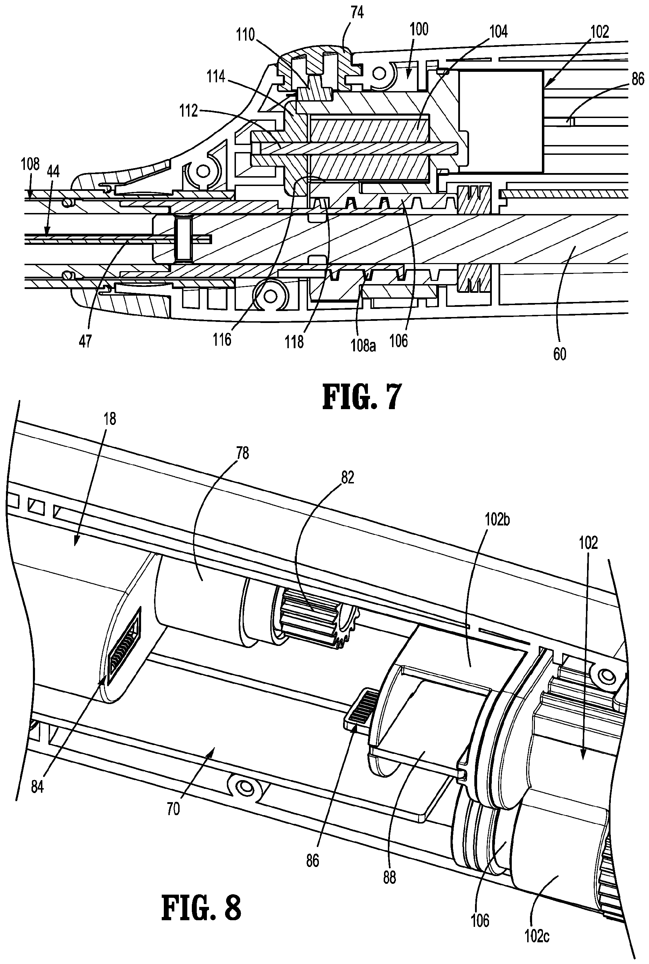

With reference to FIGS. 2, 5, 7, and 8, the instrument module 18 includes a housing 78, a motor 80 and battery (not explicitly shown) stored in the housing 78, and an output gear 82 drivingly coupled to the motor 80 and extending distally from the housing 78. In aspects, the instrument module 18 may be equipped with the processor 76 and various other electronics. The instrument module 18 is insertable into the cavity 54 of the handle housing 20 and is configured to operably couple to a fire shaft 108 provided in the handle housing 20. The instrument module 18 has a card edge power module connector 84 configured to selectively engage a card edge connector 86 of the second printed circuit board 88 in the handle housing 20 upon inserting the instrument module 18 into the handle housing 20 to electromechanically connect the fire button 74 with the motor 80 of the instrument module 18. The battery of the instrument module 18 is configured to provide power to the lights 72 via the second printed circuit board 88. In aspects, the instrument module 18 may be disposable and permanently integrated into the handle housing 20 rather than being removable and reusable.

With reference to FIGS. 2-5, 7, and 8, the handle assembly 12 includes a staple fire actuation assembly 100 configured to electromechanically fire staples from the cartridge assembly 28. The staple fire actuation assembly 100 includes a hub 102 fixed within the distal end portion 20b of the handle housing 20, a fire gear 104, a nut gear 106, and a fire shaft 108. The hub 102 has an upper portion including a left housing 102a configured to receive the output gear 82 of the instrument module 18, and a right housing 102b for supporting the fire gear 104 therein. The hub 102 further includes a collar 102c extending downwardly from the right housing 102b and disposed about the nut gear 106 to resist axial translation of the nut gear 106.

The fire gear 104 is rotationally supported in the right housing 102b of the hub 102 and is configured to operably engage the teeth of the output gear 82 of the instrument module 18, such that a rotation of the output gear 82 drives a rotation of the fire gear 104. The fire gear 104 is disposed about an axle 112 that is rotationally supported by the hub 102. The fire gear 104 may be retained in the right housing 102b by a stopper or plug 114 attached to a distal end of the axle 112.

The nut gear 106 is disposed in the lower end portion 54b of the cavity 54 and is disposed about the screw 60. The nut gear 106 has a plurality of gear teeth 116 disposed around an outer periphery of the nut gear 106. The gear teeth 116 of the nut gear 106 are in meshing engagement with the fire gear 104, and a threaded inner surface 118 of the nut gear 106 is threadingly engaged with a threaded outer surface of a proximal end portion 108a of the fire shaft 108. The fire shaft 108 is disposed about the distal end portion 60b of the screw 60 and is free to translate relative to the screw 60. The fire shaft 108 has a distal end portion 108b fixed to the pusher 34 (FIG. 4) of the cartridge assembly 28 and is configured to distally translate the pusher 34 in response to an actuation of the motor 80 of the instrument module 18.

The fire staple actuation mechanism 100 further includes a fire switch 110 in electrical communication with the instrument module 18 via the second printed circuit board 88. The fire switch 110 is disposed between the fire button 74 and the right housing 102b of the hub 102. As such, an actuation of the fire button 74 activates the motor 80 of the instrument module 18 when the instrument module 18 is connected to the second printed circuit board 88.

To assemble the handle assembly 12, the door 50 may be unlocked from the handle housing 20 and pivoted to an open position, as shown in FIG. 2. The instrument module 18, which may not be sterile, is inserted into the cavity 54 of the handle housing 20 via the proximal opening 58. Upon inserting the instrument module 18 into the handle housing 20, the card edge power module connector 84 of the instrument module 18 engages the card edge connector 86 of the second printed circuit board 88.

In use, two anatomical lumens, such as, for example, a colon, esophagus, and/or a stomach, may be disposed between the anvil plate 40 of the anvil assembly 30 and the cartridge assembly 28. With the tissue in position, a clinician manually rotates the knob 62 of the clamping actuation mechanism 52, whereby the elongated collar 64 rotates about its longitudinal axis relative to the screw 60. Due to the threaded engagement of the elongated collar 64 with the screw 60, the screw 60 translates proximally to longitudinally pull the central shaft 44 and the attached trocar 38 of the anvil assembly 30. The anvil shaft 42, which is fixed to the trocar 38, is also moved proximally along with the attached anvil head 40 from the unapproximated position to the approximated position in relation to the cartridge assembly 28.

As the screw 60 is translated, the flag 68 of the screw 60 moves relative to the sensor 66, which continuously detects the relative axial position of the flag 68. Rotation of the knob 62 is continued until the tissue is fully clamped by the end effector 16. In particular, upon the flag 68 of the screw 60 moving to a predetermined axial position relative to the sensor 66, the processor 76 activates the lights 72 of the handle assembly 12 to illuminate the fire button 74. In some aspects, the processor 76 may be disposed in the instrument module 18. With the fire button 74 illuminated, the clinician is made aware that the circular stapler 10 is ready to be fired.

An actuation of the fire button 74 causes the fire switch 110 to activate the motor 80 of the instrument module 18. Upon activating the motor 80, the output gear 82 of the instrument module 18 rotates, which drives a concomitant rotation of the fire gear 104. Due to the fire gear 104 being operably engaged with the nut gear 106, the rotation of the fire gear 104 rotates the nut gear 106, thereby driving a distal translation of the fire shaft 108 relative to the nut gear 106. The distal translation of the fire shaft 108 translates the pusher 34 distally to push staples from the cartridge assembly 28 into the clamped tissue.

While the present disclosure has been described and illustrated in connection with certain embodiments, it is not the intention of the applicant to restrict or in any other way limit the scope of the claims to such detail. Additional advantages and modifications will be readily apparent to those skilled in the art.

The invention may be described by reference to the following numbered paragraphs:-
1. A surgical stapling instrument, comprising:
   an elongated handle housing;
   a screw disposed within the handle housing and having a proximal end portion and a distal end portion;
   a rotatable knob supported by the handle housing and coupled to the proximal end portion of the screw, such that a rotation of the knob results in motion of the screw;
   an elongated body portion extending distally from the handle housing; and
   an end effector including:
      a cartridge assembly coupled to a distal end portion of the elongate body portion; and
      an anvil assembly coupled to the distal end portion of the screw and configured to translate relative to the cartridge assembly in response to a manual rotation of the knob to move the end effector between unapproximated and approximated positions.
2. The surgical stapling instrument according to paragraph 1, wherein the knob extends proximally from a proximal end portion of the handle housing.
3. The surgical stapling instrument according to paragraph 1, wherein the knob, the screw, and a proximal end portion of the elongated body portion are coaxial.
4. The surgical stapling instrument according to paragraph 1, wherein the screw is configured to translate within the handle housing in response to a rotation of the knob.
5. The surgical stapling instrument according to paragraph 1, further comprising a sensor configured to determine a relative axial position of the screw.
6. The surgical stapling instrument according to paragraph 5, wherein the screw has a flag extending outwardly therefrom and disposed adjacent the sensor, such that the sensor is configured to determine a relative axial position of the flag.
7. The surgical stapling instrument according to paragraph 5, further comprising:
   a fire switch coupled to the handle housing and in communication with a motor; and
   a light disposed adjacent the fire switch, wherein the sensor is configured to turn on the light upon determining that the screw is in an axial position corresponding to the approximated position of the end effector.
8. The surgical stapling instrument according to paragraph 1, further comprising an instrument module insertable into a cavity defined by the handle housing, the instrument module including:
   a housing;
   a motor disposed within the housing; and
   an output gear drivingly coupled to the motor.
9. The surgical stapling instrument according to paragraph 8, further comprising:
   a nut gear operably coupled to the output gear; and
   a fire shaft having a proximal end portion operably coupled to the nut gear, and a distal end portion coupled to a pusher of the cartridge assembly, wherein the fire shaft is configured to translate the pusher in response to a rotation of the nut gear.
10. The surgical stapling instrument according to paragraph 9, wherein the nut gear has a plurality of gear teeth disposed about an outer periphery thereof, and a threaded inner surface threadedly engaged to the proximal end portion of the fire shaft.
11. The surgical stapling instrument according to paragraph 10, wherein the screw extends longitudinally through the fire shaft and the nut gear.
12. The surgical stapling instrument according to paragraph 9, further comprising a fire gear intercoupling the output gear and the nut gear.
13. A handle assembly of a circular stapler, comprising:
   an elongated handle housing;
   a drive shaft disposed within the handle housing and having a proximal end portion, and a distal end portion configured to effect an opening and closing of an end effector; and
   a rotatable knob supported by the handle housing and coupled to the proximal end portion of the drive shaft, such that a rotation of the knob results in axial motion of the drive shaft.
14. The handle assembly according to paragraph 13, wherein the knob extends proximally from a proximal end portion of the handle housing.
15. The handle assembly according to paragraph 13, wherein the drive shaft is a screw disposed within a bottom end portion of the handle housing.
16. The handle assembly according to paragraph 13, further comprising a sensor disposed in the handle housing, wherein the drive shaft has a flag extending outwardly therefrom and disposed adjacent the sensor, such that the sensor is configured to determine a relative axial position of the flag.
17. The handle assembly according to paragraph 16, further comprising:
   a fire switch coupled to the handle housing and in communication with a motor; and
   a light disposed adjacent the fire switch, wherein the sensor is configured to turn on the light upon determining that the drive shaft is in an axial position corresponding to an approximated position of the end effector.
18. The handle assembly according to paragraph 13, further comprising an instrument module insertable into a cavity defined by the handle housing, the instrument module including:
   a housing;
   a motor disposed within the housing; and
   an output gear drivingly coupled to the motor.
19. The handle assembly according to paragraph 18, further comprising:
   a nut gear operably coupled to the output gear; and
   a fire shaft having a proximal end portion operably coupled to the nut gear, and a distal end portion configured to effect a stapling function of the end effector.
20. The handle assembly according to paragraph 19, wherein the drive shaft extends longitudinally through the fire shaft and the nut gear.

## Claims

1. A surgical stapling instrument, comprising:
an elongated handle housing;
a screw disposed within the handle housing and having a proximal end portion and a distal end portion;
a rotatable knob supported by the handle housing and coupled to the proximal end portion of the screw, such that a rotation of the knob results in motion of the screw;
an elongated body portion extending distally from the handle housing; and
an end effector including:
a cartridge assembly coupled to a distal end portion of the elongate body portion; and
an anvil assembly coupled to the distal end portion of the screw and configured to translate relative to the cartridge assembly in response to a manual rotation of the knob to move the end effector between unapproximated and approximated positions.

2. The surgical stapling instrument according to claim 1, wherein the knob extends proximally from a proximal end portion of the handle housing.

3. The surgical stapling instrument according to claim 1 or claim 2, wherein the knob, the screw, and a proximal end portion of the elongated body portion are coaxial.

4. The surgical stapling instrument according to any preceding claim, wherein the screw is configured to translate within the handle housing in response to a rotation of the knob.

5. The surgical stapling instrument according to any preceding claim, further comprising a sensor configured to determine a relative axial position of the screw; preferably wherein the screw has a flag extending outwardly therefrom and disposed adjacent the sensor, such that the sensor is configured to determine a relative axial position of the flag; preferably further comprising:
a fire switch coupled to the handle housing and in communication with a motor; and
a light disposed adjacent the fire switch, wherein the sensor is configured to turn on the light upon determining that the screw is in an axial position corresponding to the approximated position of the end effector.

6. The surgical stapling instrument according to any preceding claim, further comprising an instrument module insertable into a cavity defined by the handle housing, the instrument module including:
a housing;
a motor disposed within the housing; and
an output gear drivingly coupled to the motor.

7. The surgical stapling instrument according to claim 6, further comprising:
a nut gear operably coupled to the output gear; and
a fire shaft having a proximal end portion operably coupled to the nut gear, and a distal end portion coupled to a pusher of the cartridge assembly, wherein the fire shaft is configured to translate the pusher in response to a rotation of the nut gear; preferably wherein the nut gear has a plurality of gear teeth disposed about an outer periphery thereof, and a threaded inner surface threadedly engaged to the proximal end portion of the fire shaft.

8. The surgical stapling instrument according to claim 7, wherein the screw extends longitudinally through the fire shaft and the nut gear; preferably further comprising a fire gear intercoupling the output gear and the nut gear.

9. A handle assembly of a circular stapler, comprising:
an elongated handle housing;
a drive shaft disposed within the handle housing and having a proximal end portion, and a distal end portion configured to effect an opening and closing of an end effector; and
a rotatable knob supported by the handle housing and coupled to the proximal end portion of the drive shaft, such that a rotation of the knob results in axial motion of the drive shaft.

10. The handle assembly according to claim 9, wherein the knob extends proximally from a proximal end portion of the handle housing; and/or wherein the drive shaft is a screw disposed within a bottom end portion of the handle housing; preferably further comprising a sensor disposed in the handle housing, wherein the drive shaft has a flag extending outwardly therefrom and disposed adjacent the sensor, such that the sensor is configured to determine a relative axial position of the flag.

11. The handle assembly according to claim 9 or claim 10, further comprising:
a fire switch coupled to the handle housing and in communication with a motor; and
a light disposed adjacent the fire switch, wherein the sensor is configured to turn on the light upon determining that the drive shaft is in an axial position corresponding to an approximated position of the end effector.

12. The handle assembly according to any of claims 9 to 11, further comprising an instrument module insertable into a cavity defined by the handle housing, the instrument module including:
a housing;
a motor disposed within the housing; and
an output gear drivingly coupled to the motor.

13. The handle assembly according to claim 12, further comprising:
a nut gear operably coupled to the output gear; and
a fire shaft having a proximal end portion operably coupled to the nut gear, and a distal end portion configured to effect a stapling function of the end effector.

14. The handle assembly according to claim 13, wherein the drive shaft extends longitudinally through the fire shaft and the nut gear.
